# EUROPÄISCHE PATENTSCHRIFT

(11) **EP 1 485 072 B1**
(45) Veröffentlichungstag und Bekanntmachung des Hinweises auf die Patenterteilung: **29.04.2009**
(21) Anmeldenummer: 03744351.2
(22) Anmeldetag: 12.03.2003
(51) Int. Cl.: A61K 9/70, A61K 31/565, A61K 47/32

(54) **HORMONHALTIGES TRANSDERMALES THERAPEUTISCHES SYSTEM MIT EINEM WIRKSTOFFRESERVOIR AUF DER BASIS VON VINYLACETAT-VINYLPYRROLIDON-COPOLYMER MIT VERBESSERTER KOHÄSION.**
HORMONE-CONTAINING TRANSDERMAL THERAPEUTIC SYSTEM WITH AN ACTIVE SUBSTANCE RESERVOIR BASED ON VINYLACETATE-VINYLPYRROLIDONE COPOLYMER WITH IMPROVED COHESION.
SYSTEME THERAPEUTIQUE TRANSDERMIQUE CONTENANT DES HORMONES, COMPORTANT UN RESERVOIR D'AGENTS ACTIFS A BASE DE COPOLYMERE DE VINYLACETATE-VINYLPYRROLIDONE PRESENTANT UNE MEILLEURE COHESION

(30) Priorität: 16.03.2002 DE 10211832
(43) Veröffentlichungstag der Anmeldung: 15.12.2004
(73) Patentinhaber: LTS LOHMANN Therapie-Systeme AG, 56626 Andernach (DE)
(72) Erfinder: MECONI, Reinhold, 56567 Neuwied (DE); KLEIN, Robert-Peter, 56567 Neuwied (DE); SEIBERTZ, Frank, 56598 Rheinbrohl (DE); BECKER, Franz-Josef, 56567 Neuwied (DE); GÖTTE, Ursula, 53572 Unkel (DE)
(74) Vertreter: Flaccus, Rolf-Dieter
(86) Internationale Anmeldenummer: PCT/EP2003/002521
(87) Internationale Veröffentlichungsnummer: WO 2003/077890

(56) Entgegenhaltungen:
- WO-A-01/89487
- WO-A-98/02147
- WO-A-02/102390
- DE-A- 10 012 908
- DE-A- 19 613 698
- US-A- 5 919 478
- US-A- 6 007 835

## Beschreibung

Die vorliegende Erfindung betrifft transdermale therapeutische Systeme (TTS) in Pflasterform zur kontrollierten Abgabe von Sexualsteroidhormonen an die menschliche oder tierische Haut. Sie sind für therapeutische und prophylaktische Zwecke einsetzbar, sowie als Mittel zur Kontrazeption.

Estradiol, Estron (Estrogene) und Progesteron (Gestagen) sind die natürlichen weiblichen Sexualhormone. Die Sexualhormone dienen der Ausbildung der primären und sekundären Geschlechtsmerkmale. Sie haben einen Einfluß auf das Wachstum und den Körperaufbau sowie auf den Wasser- und Mineralhaushalt. Ferner prägen die Sexualhormone den Ablauf des menstruellen Zyklus bei der Frau.

Die natürlichen Sexualhormone, deren Derivate sowie Struktur-Analoga werden zur hormonalen Kontrazeption, zur Substitutions-Therapie oder zur Behandlung verschiedener Erkrankungen eingesetzt.

Ein Hauptanwendungsgebiet der Sexualhormone liegt auf dem Gebiet der postmenopausalen Hormonsubstitution. Die Substitution dient der Vorbeugung klimakterischer Beschwerden (Hitzewallungen, Schwindel, Tachykardien, Schwitzen, Angstgefühl, Reizbarkeit, Koazeatrationsschwächen, Schlafstörungen etc.). Ferner sollen Veränderungen an den Harn- und Geschlechtsorganen, kardiovaskuläre Veränderungen bedingt durch Hyperlipoproteinämien, Hautatrophien oder Osteoporose sowie weitere krankhafte Erscheinungen verhindert werden. Zu diesem Zweck wird ein Estrogen in Kombination mit einem Gestagen verabreicht.

Die transdermale Verabreichung von Wirkstoffen weist gegenüber dem oralen Verabreichungsweg eine Reihe von vorteilen auf, insbesondere eine verbesserte Bioverfügbarkeit, eine kontrollierte Abgaberate und eine Vereinfachung der Anwendung. Dies ist insbesondere bei der Hormonsubstitutionstherapie oder -prophylaxe und bei der hormonalen Kontrazeption von Bedeutung, da die Behandlung in diesen Fällen in der Regel über längere Zeiträume hinweg erfolgt.

Die transdermale Verabreichung erfolgt üblicherweise mittels transdermale therapeutischer Systeme (TTS) in Form von Wirkstoffpflastern. Der typische Aufbau eines solchen Systems umfaßt eine Rückschicht, ein damit verbundenes wirkstoffhaltiges Reservoir und eine ablösbare Schutzschicht, welche die Hautkontaktseite des Wirkstoffreservoirs während der Lagerung bzw. vor der Applikation bedeckt.

Estradiolhaltige Pflaster oder transdermale therapeutische Systeme, welche die Abgabe von Estradiol und anderer Steroidhormone an die Haut ermöglichen, sind bereits bekannt. Allerdings ist die Anzahl derartiger Applikationssysteme, die bereits auf dem Markt erhältlich sind, noch relativ gering. Die Ursache hierfür liegt darin, daß bei der Entwicklung und Herstellung hormonhaltiger Hautpflaster, welche die an sie gestellten Anforderungen zu erfüllen vermögen, verschiedene Probleme in Erscheinung treten, die bislang nur unzureichend gelöst wurden.
So muß ein hormonhaltiges Pflaster eine möglichst gleichmäßige Abgabe des Wirkstoffs über einen längeren Zeitraum ermöglichen, wobei möglichst hohe Wirkstoff-Abgaberaten (Fluxraten) durch die Haut erzielt werden müssen, um den gewünschten therapeutischen oder kontrazeptiven Effekt zu gewährleisten.

Deshalb wird bei der Entwicklung derartiger transdermaler therapeutischer Systeme im allgemeinen angestrebt, die Wirkstoffkonzentration im Wirkstoffreservoir zu erhöhen, bis die Sättigungskonzentration erreicht oder sogar überschritten wird. Auf diese Weise wird die thermodynamische Aktivität des Wirkstoffs gesteigert, wodurch eine Erhöhung der Wirkstoff-Fluxrate bewirkt wird. Allerdings hat dies auch zur Folge, daß es während der Lagerung oder während der Applikationsdauer infolge des Überschreitens der Sättigungskonzentration leicht zu einer Rekristallisation des Wirkstoffs in der Wirkstoffmatrix kommen kann. Dieses Phänomen ist insbesondere bei estradiolhaltigen Hautpflastern bekannt. Aufgrund der Rekristallisation nimmt die thermodynamische Aktivität des Wirkstoffs stark ab, und als Folge auch die Wirkstoffabgaberate. Das TTS wird dadurch instabil und entspricht nicht mehr den bestimmungsgemäßen Anforderungen. Deshalb wurden im Stand der Technik verschiedene Lösungen vorgeschlagen, mit denen hohe Wirkstoffkonzentrationen im Wirkstoffreservoir des Pflasters erreicht werden können und zugleich die Rekristallisation des Wirkstoffs verhindert werden soll.

Zur Verhinderung der Rekristallisation werden üblicherweise Hilfsstoffe eingesetzt, die ein hohes Lösungsvermögen für den betreffenden Wirkstoff, z. B. Estradiol, aufweisen. Zwar kann auf diese Weise die Rekristallisationsneigung verringert werden, jedoch wird dies mit dem Nachteil einer verringerten Freisetzung erkauft. Aufgrund der hohen Löslichkeit des Wirkstoffs in dem genannten Hilfsstoff wird der Wirkstoffflux durch die Haut vermindert.

EP 0 186 019 A1 beschreibt Wirkstoffpflaster, bei denen einer Kautschuk/Klebharzmasse in Wasser quellbare Polymere zugesetzt sind und aus denen Estradiol freigesetzt werden kann. Es hat sich jedoch gezeigt, daß die Freisetzung von Estradiol aus diesen Wirkstoffpflastern viel zu gering ist und nicht den therapeutischen Erfordernissen entspricht. In der DE-OS 39 33 460 werden Wirkstoffpflaster auf der Basis von Homo-und Copolymeren mit mindestens einem Derivat der Acryl- oder Methacrylsäure beschrieben, die zusätzlich in Wasser quellbare Substanzen enthalten sollen.

US 6 007 835 offenbart ein transdermalen Matrixsystem zur Abgabe von Estrogenen und/oder Progestogenen, dessen Matrix Vinylpyrrolidorl-Vinylacetat-Copolymer und Styrol-Isopren-Styrol-Blockcopolymer enthält.

In der Literatur sind verschiedene Substanzen beschrieben worden, welche als Kristallisationsinhibitoren wirken und die Kristallisation insbesondere von Estradiol verhindern sollen, beispielsweise Siliciumdioxid (US-Patent 5,676,968) oder wasserfreies Glycerin (WO 96/05814). Der Zusatz derartiger Stoffe ist häufig mit Nachteilen verbunden, z. B. können dadurch die mechanischen Eigenschaften (Kohäsion) des Pflasters beeinträchtigt werden, oder es treten Probleme bei der Herstellung dieser Pflaster auf.

Beilspielsweise hat sich gezeigt, daß die in DE 100 25 970 A1 beschriebenen estrogenhaltigen TTS, die durch ein Wirkstoff-reservoir auf der Basis von Ethylcellulose und einem Ethylen-Vinylacetat-Vinylpyrrolidon-Copolymer gekennzeichnet sind, eine verminderte Kohäsion aufweisen. Derartige TTS hinterlassen Rückstände auf der Haut, wenn sie nach dem Applizieren abgezogen werden. Dieselbe nachteilige Eigenschaft wurde auch bei den in DE 100 25 971 A1 offenbarten TTS bekannt.

Der vorliegenden Erfindung lag deshalb die Aufgabe zugrunde, transdermale therapeutische Systeme in Pflasterform bereitzustellen, welche für die kontrollierte Abgabe von Estrogenen und/oder Gestagenen an die menschliche oder tierische Haut geeignet sind, und bei denen die vorstehend beschriebenen Nachteile nicht auftreten.

Überraschenderweise wird diese Aufgabe dadurch gelöst, daß das Wirkstoffreservoir eines in Oberbegriff des Anspruchs 1 beschriebenen transdermalen therapeutischen Systems ein vinylpyrrolidon-vinylacetat-copolymer und die Kohäsion verbessernde Stoffe enthält, welche aus der Gruppe bestehend aus Polyethylen, Polypropylen, Salze von Ethylen-Methacrylsäure-copolymeren, Polystyrol, Polybuten, Polyisobutylen, StyrolButadien-Styrol-Blockpolymere, Polyvinylchlorid, Polyvinylidenchlorid, Polyurethan, Polyamid, Polysulfon, Polyvinylacetat, Polyvinylalkohol, Polyethylenglykol, Polyoxyethylen. Polyvinylbutyral und Ethylen-Vinylacetat-Copolymere ausgewählt sind.

Durch die Anwesenheit eines Vinylpyrrolidon-Vinylacetat-Copolymers wird die Rekristallisation der Hormon-Wirkstoffe zuverlässig verhindert. Gleichzeitig wird durch den erfindungsgemäßen Zusatz von kohäsionsverbessernden Stoffen bewirkt, daß die Wirkstoffmatrix trotz der Anwesenheit des genannten Copolymers eine ausreichende Kohäsion aufweist. Infolgedessen hinterlassen diese TTS keine Rückstände auf der Haut, wenn sie nach beendeter Applikation von der Haut abgezogen werden.

Es hat sich gezeigt, daß durch den Zusatz von Vinylpyrrolidon-Vinylacetat-Copolymer nicht nur die Rekristallisation der Wirkstoffe verhindert werden kann, sondern daß dadurch auch der Wirkstoffflux während der Applikationszeit in einem unerwarteten Maße gesteigert wird. Der Grund dafür liegt vermutlich darin, daß das Wirkstoffreservoir - aufgrund der hygroskopischen Eigenschaften des Vinylpyrrolidon-Vinylacetat-Copolymers - nach Applikation des TTS und während der Applikationsdauer Wasser (z. B. Hautfeuchtigkeit) aufnimmt, wodurch die Löslichkeit der Steroid-Wirkstoffe in der Polymermatrix des Wirkstoffreservoirs reduziert wird. Die Verminderung der Löslichkeit bewirkt einen erhöhten Wirkstoffflux durch die Haut.

Vorzugsweise enthält das wirkstoffhaltigen Reservoir 1,0 bis 95,0 Gew.-%, besonders bevorzugt 2,0 bis 90,0 Gew.-% Vinylpyrrolidon-Vinylacetat-Copolymer.
Das Verhältnis von Vinylpyrrolidon:Vinylacetat beträgt vorzugsweise 15:85 bis 85:1.5, besonders bevorzugt 20:80 bis 70:30.

Als kohäsionsverbessernde Stoffe eignen sich insbesondere Stoffe aus der nachfolgenden Gruppe: Polyethylen, Polypropylen, Salze von Ethylen-Methacrylsäure-Copolymeren (insbesondere Na- oder Zn-Salze; z. B. das Handelsprodukt Surlyn, Fa. Du Pont), Polybuten, Polyisobutylen, Polystyrol, StyrolButadien-Styrol-Blockpolymere, Styrol-Isopren-Styrol-Blockpolymere, Polyvinylchlorid, Polyvinylidenchlorid, Polyurethan, Polyamid, Polysulfon, Polyvinylacetat, Polyvinylalkohol, Polyethylenglykol, Polyoxyethylen, Polyvinylbutyral und Ethylen-Vinylacetat-Copolymere. Die letztgenannten Polymere werden besonders bevorzugt eingesetzt.

Der Gehalt an kohäsionsverbessernden Stoffen im wirkstoffhaltigen Reservoir beträgt vorzugsweise 1,0 bis 60,0 Gew.-%, besonders bevorzugt 2,0 bis 50,0 Gew.-%.

In einer bevorzugten Ausführungsform der Erfindung enthält das wirkstoffhaltige Reservoir 3,0 bis 70,0 Gew.-%, bevorzugt 5,0 bis 60,0 Gew.-% Ethylen-Vinylacetat-Copolymer.
Der Vinylacetat-Gehalt des Ethylen-vinylacetat-Copolymer beträgt vorzugsweise 15 bis 50 Gew.-%, insbesondere 18 bis 40 Gew.-%.

In der Basisvariante ist das Wirkstoffreservoir einteilig und enthält Estrogen, Gestagen, oder eine Estrogen-Gestagen-Kombination.
Gemäß einer weiteren bevorzugten Ausführungsform ist vorgesehen, daß das wirkstoffhaltige Reservoir (d. h. die Wirkstoffmatrix) aus mindestens zwei räumlich getrennten Teilen besteht. Dadurch ergeben sich mehr Möglichkeiten, um die Wirkstofffreisetzung den jeweiligen Erfordernissen anzupassen. Estrogen und Gestagen können in mindestens zwei verschiedenen, räumlich voneinander getrennten Matrices enthalten sein, die in den entsprechenden Flächengrößen beim Konfektionieren zu einem TTS zusammengefügt werden. In einer bevorzugten Ausführungsform enthält ein räumlich getrennter Teil des wirkstoffhaltigen Reservoirs ein Estrogen und der andere räumlich getrennte Teil ein Gestagen allein oder in Kombination mit einem Estrogen.
Eine weitere Variante besteht darin, zwei (oder mehrere) Reservoirs zu einem TTS zusammenzufügen, wobei eine Matrix nur einen (z. B. ein Estrogen) und die andere Matrix zwei Wirkstoffe (z. B. ein Estrogen und ein Gestagen) enthält.

Neben einem einschichtigen Aufbau des wirkstoffhaltigen Reservoirs ist gemäß weiteren bevorzugten Ausführungsformen vorgesehen, daß das Wirkstoffreservoir zwei- oder mehrschichtig aufgebaut ist. Hierdurch ergeben sich weitere Möglichkeiten für die Kontrolle der Wirkstoffabgabe. Insbesondere kann im Falle eines mehrschichtigen Aufbaus vorgesehen sein, daß die einzelnen Schichten unterschiedliche Wirkstoffe enthalten, daß sie sich hinsichtlich der Wirkstoffkonzentration unterscheiden, oder daß sie sich hinsichtlich der Zusammensetzung der Polymermatrix unterscheiden; die genannten Varianten lassen sich auch miteinander kombinieren.

Das wirkstoffhaltige Reservoir weist vorzugsweise eine Schichtdicke von 0,02 mm bis 0,5 mm auf, besonders bevorzugt von 0,03 bis 0,3 mm.

Bei weiteren bevorzugten Ausführungsformen der erfindungsgemäßen TTS ist vorgesehen, daß zwischen mindestens zwei Schichten des wirkstoffhaltigen Reservoirs ein flächenförmiges Gebilde eingelegt ist. Bei diesem flächenförmigen Gebilde handelt es sich vorzugsweise um eine Membran, eine Folie, einen textilen Stoff, einen Vliesstoff oder ein Gewebe, oder um eine Kombination solcher Materialien. Die genannten textilen Stoffe, Vliesstoffe oder Gewebe können aus Naturfasern (z. B. Cellulosefasern) oder synthetischen Fasern hergestellt sein.

Ferner kann es vorteilhaft oder erforderlich sein, das wirkstoffhaltige Reservoir mit einer haftklebenden Schicht und/oder einem haftklebenden Rand zu versehen; die genannte Schicht bzw. der Rand kann wahlweise wirkstofffrei oder wirkstoffhaltig sein.

Als Wirkstoffe kommen grundsätzlich alle natürlichen Sexualhormone, deren Derivate sowie Struktur-Analoga in Betracht, die bei der Prophylaxe bzw. Therapie menschlicher oder tierischer Krankheiten, oder zur Kontrazeption eingesetzt werden können. Vorzugsweise enthält das Wirkstoffreservoir eines erfindungsgemäßen TTS ein oder mehrere Estrogene, oder eine Kombination aus mindestens einem Estrogen und mindestens einem Gestagen. Dabei wird Estradiol (insbesondere 17-beta-Estradiol) als Estrogen besonders bevorzugt.

Ferner können insbesondere auch folgende Estrogene eingesetzt werden: 17-alpha-Estradiol, 17-beta-Estradiol-cypionat, 17-beta-Ethinylestradiol, 3,17-beta-Estradiol-dienanthat, 17-beta-Estradiolvalerate, 17-beta-Estradiol-benzoat, 17-beta-Estradiolundecylat, 17-Deacetylnorgestimat, Norgestimat, Mestranol und Quinestrol.

Als Gestagen wird Norethindronacetat (Norethisteronacetat) bevorzugt. Ferner kann/können das/die Gestagen(e) auch aus der Gruppe ausgewählt sein, die 19-Norprogesteron, Norethisteronacetat, Norethisteron, Ethisteron, Melengestrol, Norgestrel, Levonorgestrel, Gestoden, Hydroxyprogesteroncapronat, Medroxyprogesteronacetat, Ethynodioldiacetat, 17-alpha-Hydroxyprogesteron, Megestrolacetat, Lynestrenol, Desogestrel, Allylestrenol, Chlormadinon und Chlormadinonacetat umfaßt.

Die Estrogen-Konzentration im wirkstoffhaltigen Reservoir liegt vorzugsweise im Bereich von 0,5 bis 10,0 Gew.-%, besonders bevorzugt im Bereich von 1 bis 5,0 Gew.-%, und die Gestagen-Konzentration beträgt vorzugsweise 0,5 bis 20,0 Gew.-%, insbesondere 1,0 bis 10,0 Gew.-%.

Des weiteren ist vorgesehen, daß das Wirkstoffreservoir einen oder mehrere Zusatzstoffe enthält. Als Zusatzstoffe kommen insbesondere Klebharze, Weichmacher, Emulgatoren, Antioxidantien, hautpermeations-verbessernde Stoffe und Füllstoffe in Betracht. Die hierfür in Frage kommenden pharmazeutisch unbedenklichen Substanzen sind dem Fachmann grundsätzlich bekannt.

Gemäß einer bevorzugten Ausführungsform weist das Wirkstoff-Reservoir einen Gehalt an Klebharzen auf, welche die Klebrigkeit auf der Haut verbessern; dieser Gehalt liegt vorzugsweise im Bereich von 5,0 bis 70,0 Gew.-%, insbesondere im Bereich von 10,0 bis 60,0 Gew.-%.
Klebharz-Zusätze sind dem Fachmann als hydrierte Harzsäurederivate bekannt. Hydrierte Harzsäuren oder deren Derivate werden seit längerer Zeit als Grundmaterialien in Heftpflastern verwendet. Harzsäuren sind im Naturprodukt Kolophonium enthalten. Für die Herstellung der erfindungsgemäßen TTS werden bevorzugt Harzsäuren verwendet, die zum Schutz gegen Sauerstoffeinflüsse und zur Erhöhung der chemischen Inertheit partiell oder voll hydriert wurden und die zwecks Verbesserung der Alkalistabilität und ebenfalls zur Erhöhung der chemischen Inertheit an ihrer Carboxylgruppe verestert wurden. Besonders bevorzugt sind dabei Methylester, Glycerinester, Pentaerythritester, maleinsäure-modifizierte Pentaerithritester, maleinsäure-modifizierte Glycerinester oder Triethylenglycolester hydrierter Harzsäuren. Daneben können auch andere hautverträgliche Derivate des hydrierten Kolophoniums verwendet werden, sowie entsprechende Ester der nicht hydrierten Harzsäuren oder des nicht hydrierten Kolophoniums.

Um die Wirkstoffabgabe-Rate durch die Haut zu steigern, können dem Wirkstoffreservoir hautpermeationsverbessernde Stoffe hinzugefügt werden. Vorzugsweise beträgt der Gehalt dieser Stoffe im Wirkstoff-Reservoir 1,0 bis 50,0 Gew.-%, besonders bevorzugt 3,0 bis 45,0 Gew.-%.

Als Permeationsbeschleuniger (Enhancer) eignen sich beispielsweise Stoffe, die ausgewählt sind aus der Gruppe, die folgende Stoffe bzw. Stoffklassen umfaßt: gesättigte oder ungesättigte Fettsäuren, Fettsäure-Ester, insbesondere Ester mit Methanol, Ethanol oder Isopropanol (z. B. Ölsäureethylester, Ölsäuremethylester, Laurinsäuremethylester, Laurinsäureethylester, Adipinsäuremethylester, Adipinsäureethylester), geradkettige oder verzweigte Fettalkohole bzw. deren Ester, insbesondere Ester mit Essigsäure oder Milchsäure (z. B. Ethyloleat, Ethyllaurat, Ethylpalmitat, Ethyllactat, Propyllactat, Propylpalmitat, Propyllaurat, Propyloleat), mehrwertige aliphatische Alkohole oder Polyethylenglykole, Sorbitanfettsäureester und deren durch Ethoxylierung erhältlichen Derivate, Fettalkoholethoxylate, Polyoxyethylenfettsäureester; Laurinsäurediethanolamid, Ölsäurediethanolamid, Kokosfettsäurediethanolamid, D-alpha-Tocopherol, Laurinsäurehexylester, 2-Octyldodecanol, Dexpanthenol, Isopropylidenglycerol, Transcutol (= Diethylenglycol-monoethylether), DEET (= N,N-Diethyl-m-Toluolamid), Solketal, Ethanol, 1,2-Propandiol oder andere kurzkettige Alkohole (d. h. Alkohole mit bis zu 6 C-Atomen), sowie Menthol und andere ätherische Öle oder Bestandteile ätherischer Öle.
Um den Wirkstoff-Flux zu optimierten, können auch Kombinationen zweier oder mehrerer Enhancer eingesetzt werden.

Gemäß weiteren Ausführungsformen ist vorgesehen, daß das Wirkstoff-Reservoir einen oder mehrere Emulgatoren und/oder Weichmacher und/oder Antioxidantien enthält. Diese Zusatzstoffe können vorzugsweise in einer Konzentration von jeweils bis zu 25,0 Gew.-% enthalten sein, insbesondere in einer Konzentration von jeweils 1,0 bis 15,0 Gew.-%.

Als Emulgatoren können beispielsweise Polyethylenglykol, Glycerin, Natriumdodecylsulfat, Lecithin, Cetylalkohol, Cetylstearylalkohol, Sorbitanfettsäureester, PolyoxyethylenSorbitanfettsäureester, Polyoxyethylen-Fettsäureglyceride oder Polyoxyethylenfettsäureester verwendet werden.

Als Weichmacher kommen beispielsweise solche aus der Gruppe der Kohlenwasserstoffe, Alkohole (insbesondere höhere Alkohole wie Dodecanol, Undecanol, Octanol), Triglyceride, mehrwertige Alkohole, Carbonsäuren, Derivate von Carbonsäuren, Ether, Ester (z. B. Diethylphthalat, n-Butyladipat, Citronensäure-Ester) und Amine in Betracht.

Geeignete Antioxidantien sind beispielsweise Ascorbinsäure, Ascorbylpalmitat, Tocopherolacetat, Propylgallat, Butylhydroxyanisol oder Butylhydroxytoluol.

Zur Verbesserung der physikalischen Eigenschaften kann das Wirkstoffreservoir Füllstoffe enthalten, beispielsweise Titandioxid, Zinkoxid, Kreide, Aktivkohle, feinverteiltes Siliciumdioxid oder Maisstärke.

Als Rückschicht für die erfindungsgemäßen TTS eignen sich vor allem Polyester, welche sich durch besondere Festigkeit auszeichnen, wie z. B. Polyethylenterephthalat und Polybutylenterephthalat, darüber hinaus aber auch andere hautverträgliche Kunststoffmaterialien, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlichen Stoffen, die dem Fachmann bekannt sind.

Für die ablösbare Schutzfolie können dieselben Materialien verwendet werden wie für die Rückschicht, vorausgesetzt, daß diese Schicht einer geeigneten Oberflächenbehandlung, z. B. Silikonisierung oder Fluorosilikonisierung, unterzogen wird, so daß sie von der von ihr bedeckten Haftklebeschicht ablösbar ist und vor Applikation des TTS abgezogen werden kann. Es können aber auch andere ablösbare Schutzschichten, wie Polytetrafluorethylen-behandeltes Papier, Cellophan, Polyvinylchlorid, oder ähnliche verwendet werden.

Die vorliegende Erfindung betrifft ferner Verfahren zum Herstellen der erfindungsgemäßen transdermalen therapeutischen Systeme.

Gemäß einer ersten Variante des Herstellungsverfahrens wird zunächst eine Lösung oder Suspension hergestellt, welche die Matrix-Polymere, Zusatzstoffe und das/die Hormon(e) enthält. Diese Lösung oder Suspension wird mittels eines geeigneten Auftragswerks auf ein Trägermaterial oder eine geeignete Unterlage beschichtet und anschließend wird das vorhandene Lösemittel durch Trocknung entfernt. Als Unterlagen eignen sich beispielsweise Trägerfolien aus den Materialien, die oben für die Rückschicht bzw. Schutzfolie angegeben wurden.
Nach dem Entfernen der Lösemittelreste wird die erzeugte Wirkstoffreservoir-Schicht mit einer Folie (z. B. Rückschicht oder ablösbare Schutzschicht bedeckt). Zuletzt werden einzelne Wirkstoffpflaster einer bestimmten Fläche ausgestanzt und in ein adäquates Packmittel verpackt.

Ein weiteres erfindungsgemäßes Herstellungsverfahren sieht vor, daß zunächst unter Erwärmen oder Erhitzen eine Schmelze hergestellt wird, welche die Bestandteile des wirkstoffhaltigen Reservoirs enthält (Matrixpolymere, Zusatzstoffe, Steroidhormonwirkstoff(e)). Anschließend wird diese Schmelze auf eine flächenförmige Unterlage, vorzugsweise auf die genannte Rückschicht oder Schutzschicht, beschichtet und erkalten lassen. Die weitere Verarbeitung erfolgt wie oben beschrieben.

Die Herstellung der erfindungsgemäßen TTS und deren vorteilhafte Eigenschaften werden anhand der nachfolgenden Ausführungsbeispiele beschrieben.

### Beispiel 1

Unter Rühren werden zu

| | |
|---|---|
| 124,90 g | einer 20,0 %-igen Lösung von Evatane^{®} 40-55 (Fa. Atochem) in Toluol |
| 2,25 g | Estradiol-Hemihydrat und |
| 4,50 g | Norethindronacetat und |
| 35,07 g | Ethanol gegeben. Anschließend werden |
| 22,50 g | Plasdone^{®} S 630 (Fa. International Speciality Products), |
| 25,00 g | Foral^{®} 85 E (Fa. Eastman Kodak), |
| 4,53 g | Comperlan^{®} OD (Fa. Henkel), |
| 4,49 g | Cetiol^{®} A (Fa. Henkel) und |
| 1,82 g | Brij^{®} 30 (Fa. Goldschmidt) zugegeben und 135 Minuten gerührt. |

Die so erhaltene Klebmasse wird auf die Rückschicht (Hostaphan RN 23, Polyesterfolie, Fa. Mitsubishi) beschichtet, so daß nach dem Trocknen ein wirkstoffhaltiges Reservoir mit einem Flächengewicht von 60-70 g/m² resultiert. Diese Schicht wird mit der wiederablösbaren Schutzschicht (Hostaphan RN 75, einseitig silikonisiert) abgedeckt.
Aus dem so erhaltenen Laminat werden Einzelpflaster ausgestanzt.
Evatane^{®} 40-55: EVA-Copolymer mit 40 Gew.-% Vinylacetat und einem Schmelzindex von 55.
Plasdone^{®} S 630: Ethylen-Vinylacetat-Vinylpyrrolidon-Copolymer.
Foral^{®}: Klebharz (thermoplastisches Esterharz aus Kolophoniumderivaten).
Comperlan^{®} OD = Laurinsäurediethanolamid.
Cetiol^{®} A = Laurinsäurehexylester.
Brij^{®} 30 = Polyoxyethylenfettalkoholether.

### Beispiel 2

Auf die in Beispiel 1 beschriebene Weise wurden Einzelpflaster nach Beispiel 2 hergestellt, und zwar unter Verwendung folgender Zusammensetzung :

| | |
|---|---|
| 102,40 g | einer 20%-igen Lösung von Evatane^{®} 40-55 in Toluol |
| 2,25 g | Estradiol-Hemihydrat |
| 4,52 g | Norethindronacetat |
| 53,09 g | Ethanol |
| 22,50 g | Plasdone^{®}. S 630 (Fa. International Speciality Products) |
| 20,47 g | Foral^{®} 85E (Fa. Eastman Kodak) |
| 1,82 g | Brij^{®} 30 (Fa. Goldschmidt) |
| 9,02 g | Propandiol-1,2 |
| 9,02 g | Isopropylmyristat |

Zur Messung der Hautpermeation wird die Haut in eine Franzzelle eingespannt. Auf die Haut wird ein estrogen-und/oder gestagenhaltiges Pflaster mit einer Fläche von 1,539 cm² aufgeklebt und die Wirkstofffreisetzung bei 37°C gemessen (Akzeptormedium: 0,9%-ige Kochsalzlösung mit 0,1% NaN₃). Die Ergebnisse sind in der Tabelle dargestellt.

| Beispiel | Humanhautpermeation (µg/16cm²) | | | | | | | | | | | |
|---|---|---|---|---|---|---|---|---|---|---|---|---|
| | Estradiol | | | | | | Norethindronacetat | | | | | |
| | 8h | 24h | 32h | 48h | 72h | Flux | 8h | 24h | 32h | 48h | 72h | Flux |
| 1 | 49 | 218 | 318 | 474 | 653 | 0,567 | 31 | 120 | 186 | 293 | 442 | 0,418 |
| 2 | 65 | 251 | 339 | 429 | 478 | 0,296 | 49 | 155 | 221 | 312 | 414 | 0,337 |

| | | | | | | | | | | | | |
|---|---|---|---|---|---|---|---|---|---|---|---|---|
| Flux: (µg/cm²x h) | | | | | | | | | | | | |

Die Prüfung der Kohäsion wurde wie folgt durchgeführt:
Ein 16cm² großes Pflaster wurde auf den rechten Oberarm appliziert und leicht angedrückt. Nach 15 Minuten wurden die Pflaster abgezogen.
Pflaster gemäß den Beispielen 1 und 2 lassen sich einwandfrei abziehen und hinterlassen keinerlei Kleberrückstände auf der Haut.

Im Vergleich dazu verbleiben bei Mustern, die gemäß den Beispielen 1, 2 und 3 der DE 100 25 970 hergestellt wurden, erhebliche Kleberrückstände auf der Haut.

Die erfindungsgemäßen transdermalen therapeutischen Systeme eignen sich in vorteilhafter Weise für therapeutische Zwecke in der Humanmedizin, vorzugsweise zur Hormonsubstitution, zur Prophylaxe und Behandlung klimakterischer Beschwerden, sowie zur Osteoporose-Prophylaxe und für die hormonale Kontrazeption. Die vorliegende Erfindung schließt deshalb die Verwendung der erfindungsgemäßen TTS in den genannten therapeutischen Verfahren sowie zur Kontrazeption mit ein.

## Patentansprüche

1. Transdermales therapeutisches System in Pflasterform zur kontrollierten Abgabe von Estrogenen und/oder Gestagenen an die menschliche oder tierische Haut, wobei das System eine Rückschicht, ein damit verbundenes, ein- oder mehrschichtiges wirkstoffhaltiges Reservoir und eine wiederablösbare Schutzschicht aufweist, **dadurch gekennzeichnet, daß** das wirkstoffhaltige Reservoir Vinylpyrrolidon-Vinylacetat-Copolymer und die Kohäsion verbessernde Stoffe enthält, wobei die kohäsionsverbesseraden Stoffe ausgewählt sind aus der Gruppe bestehend aus Polyethylen, Polypropylen, Salze von Ethylen-Methacrylsäure-Copolymeren, Polystyrol, Polybuten, Polyisobutylen, Styrol-Butadien-Styrol-Blockpolymere, Polyvinylchlorid, Polyvinylidenchlorid, Polyurethan, Polyamid, Polysulfon, Polyvinylacetat, Polyvinylalkohol, Polyethylenglykol, Polyoxyethylen, Polyvinylbutyral und Ethylen-Vinylacetat-Copolymere.

2. Transdermales therapeutisches System nach Anspruch 1, **dadurch gekennzeichnet, daß** der Gehalt an Vinylpyrrolidon-Vinylacetat-Copolymer im wirkstoffhaltigen Reservoir 1,0 bis 95,0 Gew:-%, bevorzugt 2,0 bis 90,0 Gew.-% beträgt.

3. Transdermales therapeutisches System nach Anspruch 1 oder 2, **dadurch gekennzeichnet, daß** das Vinylpyrrolidon/Vinylacetat-Verhältnis des Vinylpyrrolidon-Vinylacetat-Copolymeren 15:85 bis 85:15, bevorzugt 20:80 bis 70:30 beträgt.

4. Transdermales therapeutisches System nach einem oder mehreren der vorangehenden Ansprüche, **dadurch gekennzeichnet, daß** der Gehalt an kohäsionsverbessernden Stoffen im wirkstoffhaltigen Reservoir 1,0 bis 60,0 Gew.-%, bevorzugt 2,0 bis 50,0 Gew.-% beträgt.

5. Transdermales therapeutisches System nach einem oder mehreren der vorangehenden Ansprüche, **dadurch gekennzeichnet, daß** das wirkstoffhaltige Reservoir 3,0 bis 70,0 Gew.-%, bevorzugt 5,0 bis 60,0 Gew.-% Ethylen-Vinylacetat-Copolymer enthält.

6. Transdermales therapeutisches System nach Anspruch 5, **dadurch gekennzeichnet, daß** der Vinylacetat-Gehalt des Ethylen-Vinylacetat-Copolymer 15 bis 50 Gew.-%, bevorzugt 18 bis 40 Gew.-% beträgt.

7. Transdermales therapeutisches System nach einem oder mehreren der vorangehenden Ansprüche, **dadurch gekennzeichnet, daß** das wirkstoffhaltige Reservoir aus mindestens zwei räumlich getrennten Teilen besteht.

8. Transdermales therapeutisches System nach Anspruch 7, **dadurch gekennzeichnet, daß** ein räumlich getrennter Teil des wirkstoffhaltigen Reservoirs ein Estrogen und der andere räumlich getrennte Teil ein Gestagen allein oder in Kombination mit einem Estrogen enthält.

9. Transdermalen therapeutisches System nach einem oder mehreren der vorangehenden Ansprüche, **dadurch gekennzeichnet, daß** die Estrogenkonzentration im wirkstoffhaltigen Reservoir 0,5 bis 10,0 Gew.-%, bevorzugt 1 bis 5,0 Gew.-% und die Gestagen-Konzentration 0,5 bis 20,0 Gew.-%, bevorzugt 1,0 bis 10,0 Gew.-%-beträgt.

10. Transdermales therapeutisches System nach einem oder mehreren der vorangehenden Ansprüche, **dadurch gekennzeichnet, daß** das wirkstoffhaltige Reservoir die Klebrigkeit verbessernde Harze in einer Konzentration von 5,0 bis 70,0 Gew.-%, bevorzugt 10,0 bis 60,0 Gew.-% enthält.

11. Transdermales therapeutisches System nach einem oder mehreren der vorangehenden Ansprüche, **dadurch gekennzeichnet, daß** das wirkstoffhaltige Reservoir die Hautpermeation steigernde Stoffe in einer Konzentration von 1,0 bis 50,0 Gew.-%, bevorzugt 3,0 bis 45,0 Gew.-% enthält.

12. Transdermales therapeutisches System nach einem der vorangehenden Ansprüche, **dadurch gekennzeichnet, daß** das wirkstoffhaltige Reservoir Emulgatoren und/oder Weichmacher und/oder Antioxidantien in einer Konzentration von jeweils bis zu 25,0 Gew.-%, bevorzugt 1, 0 bis 15,0 Gew.-% enthält.

13. Transdermales therapeutisches System nach einem der vorangehenden Ansprüche, **dadurch gekennzeichnet, daß** das wirkstoffhaltige Reservoir Füllstoffe enthält.

14. Transdermalen, therapeutisches System nach einem der vorangehenden Ansprüche, **dadurch gekennzeichnet, daß** das wirkstoffhaltige Reservoir aus zwei oder mehreren Schichten aufgebaut ist.

15. Transdermales therapeutisches System nach Anspruch 14, **dadurch gekennzeichnet, daß** das die einzelnen Schichten des wirkstoffhaltigen Reservoirs unterschiedliche Wirkstoffe enthalten und/oder sich hinsichtlich der Wirkstoffkonzentration und/oder der Zusammensetzung unterscheiden.

16. Transdermales therapeutisches System nach Anspruch 14 oder 15, **dadurch gekennzeichnet, daß** zwischen den Schichten des wirkstoffhaltigen Reservoirs ein flächenförmiges Gebilde, vorzugsweise eine Membran, eine Folie, ein textiler Stoff, ein Vliesstoff oder ein Gewebe, eingelegt ist.

17. Transdermales therapeutisches System nach einem der vorangehenden Ansprüche, **dadurch gekennzeichnet, daß** das wirkstoffhaltige Reservoir eine Schichtdicke von 0,02 mm bis 0,5 mm, bevorzugt 0,03 mm bis 0,3 mm aufweist.

18. Transdermales therapeutisches System nach einem der vorangehenden Ansprüche, **dadurch gekennzeichnet, daß** das wirkstoffhaltige Reservoir mit einer haftklebenden Schicht und/oder einem haftklebenden Rand versehen ist.

19. Verfahren zur Herstellung eines transdermalen therapeutischen Systems nach einem der vorangehenden Ansprüche, umfassend die folgenden Schritte:
- Herstellung einer Lösung oder Suspension, welche die Bestandteile des wirkstoffhaltigen Reservoirs enthält, und
- Beschichtung dieser Lösung oder Suspension auf eine flächenförmige Unterlage.

20. Verfahren zur Herstellung eines transdermalen therapeutischen Systems nach einem der vorangehenden Ansprüche, umfassend die folgenden Schritte:
- Herstellung einer Schmelze, welche die Bestandteile des wirkstoffhaltigen Reservoirs enthält, und
- Beschichtung dieser Schmelze auf eine flächenförmige Unterlage.

## Claims

1. Transdermal therapeutic system in the form of a plaster, for the controlled delivery of estrogens and/or gestagens to human or animal skin, said system having a backing layer, an active substance-containing, mono- or multilayer reservoir connected thereto, and a detachable protective layer, **characterized in that** the active substance-containing reservoir contains vinyl pyrrolidone-vinyl acetate copolymer and cohesion-enhancing substances, said cohesion-enhancing substances being selected from the group consisting of polyethylene, polypropylene, salts of ethylene-methacrylic acid copolymers, polystyrene, polybutene, polyisobutylene, styrene-butadiene-styrene block polymers, polyvinyl chloride, polyvinylidene chloride, polyurethane, polyamide, polysulfone, polyvinyl acetate, polyvinyl alcohol, polyethylene glycol, polyoxyethylene, polyvinyl butyral, and ethylene-vinyl acetate copolymers.

2. Transdermal therapeutic system according to claim 1, **characterized in that** the content of vinyl pyrrolidone-vinyl acetate copolymer in the active substance-containing reservoir is 1.0 to 95.0%-wt, preferably 2.0 to 90.0%-wt.

3. Transdermal therapeutic system according to claim 1 or 2, **characterized in that** the vinyl pyrrolidone/vinyl acetate ratio of the vinyl pyrrolidone-vinyl acetate copolymer is 15:85 to 85:15, preferably 20:80 to 70:30.

4. Transdermal therapeutic system according to one or more of the preceding claims, **characterized in that** the content of cohesion-improving substances in the active substance-containing reservoir is 1.0 to 60.0%-wt, preferably 2.0 to 50.0%-wt.

5. Transdermal therapeutic system according to one or more of the preceding claims, **characterized in that** the active substance-containing reservoir contains 3.0 to 70.0%-wt, preferably 5.0 to 60.0%-wt, of ethylene-vinyl acetate copolymer.

6. Transdermal therapeutic system according to claim 5, **characterized in that** the vinyl acetate content of the ethylene-vinyl acetate copolymer is 15 to 50%-wt, preferably 18 to 40%-wt.

7. Transdermal therapeutic system according to one or more of the preceding claims, **characterized in that** the active substance-containing reservoir consists of at least two spatially separated parts.

8. Transdermal therapeutic system according to claim 7, **characterized in that** one spatially separated part of the active substance-containing reservoir contains an estrogen, and that the other spatially separated part contains a gestagen, either alone or in combination with an estrogen.

9. Transdermal therapeutic system according to one or more of the preceding claims, **characterized in that** the estrogen concentration in the active substance-containing reservoir is 0.5 to 10.0%-wt, preferably 1 to 5.0%-wt, and that the gestagen concentration is 0.5 to 20.0%-wt, preferably 1.0 to 10.0%-wt.

10. Transdermal therapeutic system according to one or more of the preceding claims, **characterized in that** the active substance-containing reservoir contains tack-enhancing resins in a concentration of 5.0 to 70.0%-wt, preferably 10.0 to 60.0%-wt.

11. Transdermal therapeutic system according to one or more of the preceding claims, **characterized in that** the active substance-containing reservoir contains skin permeation-enhancing substances in a concentration of 1.0 to 50.0%-wt, preferably 3.0 to 45.0%-wt.

12. Transdermal therapeutic system according to one of the preceding claims, **characterized in that** the active substance-containing reservoir contains emulsifiers and/or plasticizers and/or antioxidants in a respective concentration of up to 25.0%-wt, preferably 1.0 to 15.0%-wt.

13. Transdermal therapeutic system according to one of the preceding claims, **characterized in that** the active substance-containing reservoir contains fillers.

14. Transdermal therapeutic system according to one of the preceding claims, **characterized in that** the active substance-containing reservoir is made up of two or more layers.

15. Transdermal therapeutic system according to claim 14, **characterized in that** the individual layers of the active substance-containing reservoir contain different active substances and/or differ from each other in terms of their active substance concentration and/or their composition.

16. Transdermal therapeutic system according to claim 14 or 15, **characterized in that** between the layers of the active substance-containing reservoir there is interposed a flat body, preferably a membrane, a film, a textile fabric, a nonwoven fabric, or a woven fabric.

17. Transdermal therapeutic system according to any one of the preceding claims, **characterized in that** the active substance-containing reservoir has a layer thickness of 0.02 mm to 0.5 mm, preferably 0.03 to 0.3 mm.

18. Transdermal therapeutic system according to any one of the preceding claims, **characterized in that** the active substance-containing reservoir is provided with a pressure sensitive adhesive layer and/or a pressure-sensitive adhesive margin.

19. Process for the production of a transdermal therapeutic system according to any one of the preceding claims, comprising the following steps:
- preparing a solution or suspension containing the components of the active substance-containing reservoir, and
- coating this solution or suspension on a flat support.

20. Process for the production of a transdermal therapeutic system according to any one of the preceding claims, comprising the following steps:
- preparing a melt containing the components of the active substance-containing reservoir, and
- coating this melt on a flat support.

## Revendications

1. Système thérapeutique transdermique sous forme d'emplâtres pour la distribution contrôlée d'oestrogènes et/ou de gestagènes sur la peau humaine ou animale, le système présentant une couche dorsale, un réservoir monocouche ou multicouche qui lui est relié contenant une ou plusieurs substances actives, et une couche de protection repositionnable, **caractérisé en ce que** le réservoir contenant une ou plusieurs substances actives contient un copolymère de vinylpyrrolidone-acétate de vinyle et des substances améliorant la cohésion, les substances améliorant la cohésion étant choisies parmi le groupe constitué par du polyéthylène, du polypropylène, des sels de copolymères d'éthylène-acide méthacrylique, du polystyrène, du polybutène, du polyisobutylène, des copolymères séquencés de styrène-butadiène-styrène, du chlorure de polyvinyle, du chlorure de polyvinylidène, du polyuréthane, du polyamide, de la polysulfone, de l'acétate de polyvinyle, de l'alcool polyvinylique, du polyéthylèneglycol, du polyoxyéthylène, du polyvinylbutyral et des copolymères d'éthylène-acétate de vinyle.

2. Système thérapeutique transdermique selon la revendication 1, **caractérisé en ce que** la teneur en copolymère de vinylpyrrolidone-acétate de vinyle du réservoir contenant une ou plusieurs substances actives s'élève de 1,0 à 95,0 % en poids, de préférence de 2,0 à 90,0 % en poids.

3. Système thérapeutique transdermique selon la revendication 1 ou 2, **caractérisé en ce que** la proportion vinylpyrrolidone/acétate de vinyle du copolymère de vinylpyrrolidone-acétate de vinyle s'élève de 15:85 à 85:15, de préférence de 20:80 à 70:30.

4. Système thérapeutique transdermique selon une ou plusieurs des revendications précédentes, **caractérisé en ce que** la teneur en substances améliorant la cohésion du réservoir contenant une ou plusieurs substances actives s'élève de 1,0 à 60,0 % en poids, de préférence de 2,0 à 50,0 % en poids.

5. Système thérapeutique transdermique selon une ou plusieurs des revendications précédentes, **caractérisé en ce que** le réservoir contenant une ou plusieurs substances actives contient un copolymère d'éthylène-acétate de vinyle à concurrence de 3,0 à 70,0 % en poids, de préférence de 5,0 à 60,0 % en poids.

6. Système thérapeutique transdermique selon la revendication 5, **caractérisé en ce que** la teneur en acétate de vinyle du copolymère d'éthylène-acétate de vinyle s'élève de 15 à 50 % en poids, de préférence de 18 à 40 % en poids.

7. Système thérapeutique transdermique selon une ou plusieurs des revendications précédentes, **caractérisé en ce que** le réservoir contenant une ou plusieurs substances actives est constitué par au moins deux parties séparées l'une de l'autre dans l'espace.

8. Système thérapeutique transdermique selon la revendication 7, **caractérisé en ce que** une partie séparée dans l'espace du réservoir contenant une ou plusieurs substances actives contient un oestrogène et l'autre partie séparée dans l'espace contient un gestagène, seul ou en combinaison avec un oestrogène.

9. Système thérapeutique transdermique selon une ou plusieurs des revendications précédentes, **caractérisé en ce que** la concentration de l'oestrogène dans le réservoir contenant une ou plusieurs substances actives s'élève de 0,5 à 10,0 % en poids, de préférence de 1 à 5,0 % en poids, et la concentration du gestagène s'élève de 0,5 à 20,0 % en poids, de préférence de 1,0 à 10,0 % en poids.

10. Système thérapeutique transdermique selon une ou plusieurs des revendications précédentes, **caractérisé en ce que** le réservoir contenant une ou plusieurs substances actives contient des résines améliorant l'adhésivité en une concentration de 5,0 à 70,0 % en poids, de préférence de 10,0 à 60,0 % en poids.

11. Système thérapeutique transdermique selon une ou plusieurs des revendications précédentes, **caractérisé en ce que** le réservoir contenant une ou plusieurs substances actives contient des substances augmentant la perméation cutanée en une concentration de 1,0 à 50,0 % en poids, de préférence de 3,0 à 45,0 % en poids.

12. Système thérapeutique transdermique selon une ou plusieurs des revendications précédentes, **caractérisé en ce que** le réservoir contenant une ou plusieurs substances actives contient des émulsifiants et/ou des plastifiants et/ou des antioxydants en une concentration s'élevant respectivement jusqu'à 25,0 % en poids, de préférence de 1,0 à 15,0 % en poids.

13. Système thérapeutique transdermique selon une ou plusieurs des revendications précédentes, **caractérisé en ce que** le réservoir contenant une ou plusieurs substances actives contient des substances de charge.

14. Système thérapeutique transdermique selon une ou plusieurs des revendications précédentes, **caractérisé en ce que** le réservoir contenant une ou plusieurs substances actives est constitué par deux couches ou plus.

15. Système thérapeutique transdermique selon la revendication 14, **caractérisé en ce que** les couches individuelles du réservoir contenant une ou plusieurs substances actives contiennent des substances actives différentes et/ou se distinguent en ce qui concerne la concentration de la ou des substance(s) active(s) et/ou la composition.

16. Système thérapeutique transdermique selon la revendication 14 ou 15, **caractérisé en ce que**, entre les couches du réservoir contenant une ou plusieurs substances actives, vient s'insérer un produit plat, de préférence une membrane, une feuille mince, une matière textile, un non-tissé ou un tissu.

17. Système thérapeutique transdermique selon une ou plusieurs des revendications précédentes, **caractérisé en ce que** le réservoir contenant une ou plusieurs substances actives présente une épaisseur de couche de 0,02 mm à 0,5 mm, de préférence de 0,03 mm à 0,3 mm.

18. Système thérapeutique transdermique selon une ou plusieurs des revendications précédentes, **caractérisé en ce que** le réservoir contenant une ou plusieurs substances actives est muni d'une couche adhésive et/ou d'un bord adhésif.

19. Procédé pour la fabrication d'un système thérapeutique transdermique selon l'une quelconque des revendications précédentes, comprenant les étapes suivantes :
- la préparation d'une solution ou d'une suspension qui contient les constituants du réservoir contenant une ou plusieurs substances actives, et
- l'enduction d'un substrat de forme plate à l'aide de cette solution ou de cette suspension.

20. Procédé pour la fabrication d'un système thérapeutique transdermique selon l'une quelconque des revendications précédentes, comprenant les étapes suivantes :
- la préparation d'une masse fondue qui contient les constituants du réservoir contenant une ou plusieurs substances actives, et
- l'enduction d'un substrat de forme plate à l'aide de cette masse fondue.
